# EUROPEAN PATENT SPECIFICATION

(11) **EP 3 068 381 B1**
(45) Date of publication and mention of the grant of the patent: **11.09.2019**
(21) Application number: 14798839.8
(22) Date of filing: 13.11.2014
(51) Int. Cl.: A61K 9/48, A61K 9/16

(54) **MULTI-PARTICULATE DRUG DELIVERY SYSTEM**
MULTIPARTIKULÄRES ARZNEIMITTELABGABESYSTEM
SYSTÈME D'ADMINISTRATION DE MÉDICAMENT MULTIPARTICULAIRE

(30) Priority: 13.11.2013 EP 13192657; 03.03.2014 EP 14157427
(43) Date of publication of application: 21.09.2016
(73) Proprietor: Tillotts Pharma AG, 4310 Rheinfelden (CH)
(72) Inventor: BRAVO GONZALÉZ, Roberto Carlos, 4102 Binningen (CH); VARUM, Felipe José Oliveira, CH-4057 Basel (CH); DE KRUIF, Jan, Kendall, F-68300 Saint-Louis (FR); KUENTZ, Martin, CH-4132 Muttenz (CH)
(74) Representative: Teipel, Stephan
(86) International application number: PCT/EP2014/074501
(87) International publication number: WO 2015/071366

(56) References cited:
- EP-A1- 1 475 070
- WO-A2-2007/129926
- RAMPURNA PRASAD GULLAPALLI: "Soft gelatin capsules (softgels)", JOURNAL OF PHARMACEUTICAL SCIENCES, vol. 99, no. 10, 18 October 2010 (2010-10-18), pages 4107-4148, XP055090285, ISSN: 0022-3549, DOI: 10.1002/jps.22151
- DAE KUN HWANG ET AL: "Stop-Flow Lithography for the Production of Shape-Evolving Degradable Microgel Particles", JOURNAL OF THE AMERICAN CHEMICAL SOCIETY, vol. 131, no. 12, 1 April 2009 (2009-04-01), pages 4499-4504, XP055156286, ISSN: 0002-7863, DOI: 10.1021/ja809256d
- "The Merck index; Petrolatum ED - O'Neil M J; Smith A; Heckelman P E; ET AL (Editors)", 1 January 2001 (2001-01-01), THE MERCK INDEX : AN ENCYCLOPEDIA OF CHEMICALS, DRUGS, AND BIOLOGICAL 13TH ED, MERCK & CO, WHITEHOUSE STATION, NJ, USA, PAGE(S) 1289, XP002732108, ISBN: 978-0-911910-13-1

## Description

The present invention relates to a capsule filled with a multi-particulate drug delivery system and a process for its preparation.

Oral delivery of active pharmaceutical ingredients (in the following abbreviated as API) is an important research field in pharmaceutical technology. On the way to the site of therapeutic activity for local-acting APIs as well as to the site of drug absorption for systemically exposed compounds, the bioavailability of APIs is compromised by several barriers. It starts with the luminal instability of a number of APIs in the harsh conditions of the gastro-intestinal tract, particularly in the stomach. Thus, a delivery system has to cope with acidic and enzymatic barrier to bring APIs intact to the site of absorption or of local action. Another substantial hurdle is the permeation step through the gut wall. In particular, big molecules are too bulky to be passively absorbed through the intestinal wall. Other ways of absorbing would have to be used such as paracellular transport, transcytosis or uptake by the intestinal M-cells. Some APIs have therapeutic action locally in the gastrointestinal lumen, in the mucosa, either binding to specific cell receptors or to cytokines produced by the epithelium. In these cases, the hurdles related to the systemic exposure through the gastrointestinal mucosa are of benefit for locally acting large molecules. In both events, i.e. systemically exposed or locally acting APIs, a common challenge is their delivery to the site of action without compromising their biological activity.

Among various options for protecting and delivering APIs to their site of action within the gastrointestinal tract after oral administration, a lipid-based drug delivery can be envisaged. However, a standard lipid based system is not able to target a specific region of the gut. Furthermore, one of the technical challenges is that an aqueous environment would be required for many APIs. A hydrophilic micro-environment might be obtained by inverse microemulsion or liposomes. A basic issue of using liposomes or W/O microemulsions is that upon dilution in the gastrointestinal tract, there are phase changes taking place leading to colloidal instability. Moreover, these lipid-based formulations are digested by the lipophilic enzymes including the phospholipase A2, which degrades liposomes and other phospholipid-based systems. Therefore, a more stable hydrophilic compartment would be desirable for drug inclusion.

One option for including an API in a hydrophilic compartment is microencapsulation. Many different techniques for the production of microspheres and microcapsules have been described. An overview over these techniques is provided by M. Whelehan, et al., in Journal of Microencapsulation, 2011; 28(8): 669-688. The vibrating nozzle technique is a widely used method for the production of microspheres and microcapsules. This technique is for example disclosed in WO 2009/130225 and by M. Homar, et al., in Journal of Microencapsulation, February 2007; 24(1): 72-81, C.-Y. Yu, et al., in Journal of Microencapsulation, 2010; 27(2): 171-177, H. Brandenberger, et al., in Journal of Biotechnology 63 (1998) 73-80 and G. Auriemma, et al., in Carbohydrate Polymers 92 (2013) 367-373.

A disadvantage of the known approaches is that the obtained polymer particles need to be gelled in order to solidify the particles. This gelling is generally accomplished by ionic gelation in the presence of dissolved divalent or trivalent metal ions, such as Ca²⁺. For example, droplets of a sodium alginate solution fall into a hardening bath containing a solution of CaCl₂ to gel the droplets forming Ca-alginate in a rapid ionotropic reaction. However, up to now, aqueous solutions of the divalent and trivalent salts were used as hardening baths. Therefore, the obtained dispersion of the microparticles in the aqueous hardening bath is not suitable for example for being directly filled into gelatin capsules because the water present in the aqueous phase would soften or even dissolve the capsule shell. Consequently, the gelled microparticles have to be collected and dried prior to further processing into unit doses. Furthermore, the storage stability of the microparticles in particular in the aqueous hardening bath is low. This requires collecting and drying of the microparticles immediately after their precipitation. Finally, the encapsulation efficiency by hardening the microparticles in an aqueous bath is low.

WO 2007/129926 discloses a method to encapsulate bioactive macromolecules into polymeric particles by an emulsification/internal gelation procedure comprising the formation of a water-in-oil emulsion followed by solubilization of dispersed insoluble calcium complex triggering gelation of said polymer dispersed in the internal phase. The resulting gelled particles dispersed in the oil phase are recovered by partition phases coupled with high speed centrifugation cycles. The water-in-oil emulsion is prepared by mechanical stirring. As external oil phase a mixture if paraffin oil and sorbitan monooleate is used. The obtained polymeric particles have a size of less than 10 µm in diameter.

EP-A-1 475 070 discloses a water-in-oil emulsion composition comprising a microgel obtained by dissolving a hydrophilic compound having a gelation ability in water or an aqueous component, letting it cool down and solidify to form a gel, and pulverizing said gel.

The scientific article "Soft Gelatin Capsules (Softgels)" published in Journal of Pharmaceutical Sciences, Vol. 99, No. 10, October 2010, pages 4107-4147, of Gullapalli refers to the advantages of soft dosage forms over other oral dosage forms

In the scientific article "Stop-flow Lithography for the Production of Shape-Evolving Degradable Microgel Particles" published in J. Am. Chem. Soc., 2009, 131, pages 4499-4504, Hwang et al. relate to the synthesis of microgel particles capable of bulk degradation by using stop-flow lithography.

Therefore, there is still a need for further multi-particulate drug delivery systems which overcome the above problems and which can be prepared more easily in a more cost efficient manner. In particular, there is a need for multi-particulate drug delivery systems which are suitable for being directly filled into a capsule for example into gelatin, hydroxypropylmethylcellulose (HPMC), or other types of capsules without the requirement of intermediate separation and drying steps.

It has now surprisingly been found that microgel particles can be obtained in a non-aqueous, in particular lipid hardening bath. This allows using the obtained dispersion of the microgel particles in the hardening bath in the preparation of the capsule according to the claimed invention without the requirement of an intermediate separation and drying of the microgel particles. Furthermore, it was surprisingly found that, by using a non-aqueous hardening bath, the encapsulation efficiency is significantly increased and the thereby obtained microgel particles have an increased stability even during prolonged storage in the hardening bath.

Thus, the present invention relates to a capsule containing a multi-particulate drug delivery system which comprises microgel particles containing an active pharmaceutical ingredient, said microgel particles being dispersed in a liquid non-aqueous composition which does not contain any paraffin oil.

In the context of the present invention "microgel particles" denote microparticles formed of a gel (microgels). The microgel particles are preferably such which are obtainable by the vibrating nozzle technique (also called "prilling"). In particular, the microgel particles are such which are not obtainable by an emulsification process. The microgels are dispersed in a liquid non-aqueous composition. The drug delivery system can therefore also be referred to as dispersion, preferably lipid-based dispersion of microgels.

The term "multi-particulate" is to be understood as denoting a plurality of individual particles which may be of the same or different type as will be explained in more detail below.

In a preferred embodiment, the non-aqueous composition only contains pharmaceutically acceptable ingredients. Thus, the composition preferably does not contain any toxic organic solvents, such as n-hexane or n-butanol. Suitable solvents in the non-aqueous composition are, for example, pharmaceutically acceptable alcohols, such as ethanol, diethylene glycol monoethylether and lipids. Suitable lipid compositions will be explained in more detail below. According to the claimed invention, the non-aqueous composition does not contain any paraffin oil.

The multi-particulate drug delivery system of the capsule of the present invention is suitable for delivering an API to its site of pharmacological action or absorption upon oral administration. Thereby the microgel particles protect the API for example against enzymatic degradation and the polymer in the microgel particles can for example be selected such that it provides mucoadhesion in order to further facilitate the local action or systemic absorption of the API. A further advantage of the multi-particulate drug delivery system of the capsule of the present invention is that the microgel particles are dispersed in a liquid non-aqueous, in particular lipid composition, which does not contain any paraffin oil, so that the system can for example be contained in a gelatin capsule for oral administration.

The API contained in the microgel particles is not limited to specific physiochemical properties. The API can be hydrophilic or hydrophobic. However, if the microgel particles are prepared using an aqueous solution of a polymer, hydrophilic APIs are preferred. The microgel particles can contain one or more APIs either in pure form or for example in the form of vesicles containing the API. The multi-particulate drug delivery system of the capsule of the present invention is particularly suitable for bulky API molecules which are otherwise difficult to be transported to their sites of pharmacological action upon oral administration. In particular for bulky API molecules it is difficult to maintain a favorable environment during their transport through the gastrointestinal tract in order to preserve their biological activity. This problem is successfully solved by the present invention.

Furthermore, pharmaceutical proteins and peptides are becoming an important class of therapeutic drugs. However, due to their large molecular weight and size, they show poor permeability characteristics through various mucosal surfaces and biological membranes. Moreover, their inherent chemical and physical instability are also factors which result in the low bioavailability associated with the oral delivery. A further advantage of the multi-particulate drug delivery system of the present invention is that since the microgel particles usually provide a hydrophilic environment proteins and peptides which are usually also hydrophilic, can be dissolved in the microgel particles, thus being readily available at target site. Furthermore, the microgel particles can successfully protect peptides and proteins from the gastrointestinal tract environment. Therefore, proteins and peptides are preferred APIs in the multi-particulate drug delivery system of the present invention.

The microgel particles can be in the form of beads containing the gelled polymer throughout the particles forming a matrix for the API or in the form of microcapsules comprising a core containing the API and a shell formed of the gelled polymer.

The microgel particles can have any suitable size. The size of the particles can for example be in the range of 1 to 2.000 µm, preferably 10 to 2.000 µm or 20 to 2.000 µm, more preferably in the range of 50 to 1.000 µm, and even more preferably in the range of 80 to 500 µm. In one embodiment the particle size distribution expressed by the 90th percentiles D₉₀ can be below 1000 µm, such as below 700 µm and preferably below 500 µm. Preferably, the particle size distribution D₉₀ is above 10 µm, more preferably above 20 µm. The particle size distribution D₉₀ can be in the range of 10 to 1000 µm, preferably in the range of 100 to 700 µm and more preferably in the range of 250 to 500 µm. In another embodiment the particle size distribution expressed by the median particle size D₅₀ can be below 1.000 µm, such as below 700 µm and preferably below 500 µm. Preferably, the median particle size D₅₀ can be above 10 µm, more preferably above 20 µm. The median particle size D₅₀ can be in the range of 10 to 1.000 µm, preferably in the range of 100 to 700 µm and more preferably in the range of 250 to 500 µm. In a preferred embodiment the particle size distribution satisfies both criteria, the above D₉₀ values and the above D₅₀ values.

Furthermore, the microgel particles can have any suitable form. For example, the particles can be spherical or non-spherical, like elliptic. Furthermore, the particles may exhibit a toroidal shape which resembles that of erythrocytes. The particle shape can be described by the elongation factor, which is the max Feret diameter (the linear segment connecting the two perimeter points that are the furthest apart) divided by the Feret equivalent rectangular short side (the shortest side of the rectangle with the same area as the particle and the longest side equal in length to the max Feret diameter). Preferably, the elongation factor of the particles is in the range of 1.27 to 2.60, more preferably in the range of 1.27 to 2.30, and even more preferably in the range of 1.60 to 2.20.

In this regard, it was surprisingly found that when microgel particles are prepared according to the prior art using an aqueous hardening bath the elongation factor of the obtained particles is above 2.8 and in particular is about 2.9. By using a non-aqueous hardening bath, microgel particles having a lower elongation factor can be obtained. Thusthe microgel particles used in the claimed invention contain at least one gel-forming polymer having a particle size distribution D₉₀ of below 1000 µm, preferably of below 700 µm and more preferably of below 500 µm, and having an elongation factor in the range of 1.27 to 2.60, preferably in the range of 1.27 to 2.30, and even more preferably in the range of 1.60 to 2.20. In a further embodiment the microgel particles can have a median particle size D₅₀ of below 1.000 µm, preferably of below 700 µm and more preferably of below 500 µm. These microgel particles may contain the same gel-forming polymers as the microgel particles in the multi-particulate drug delivery system of the capsule of the invention.

The above described size and form of the microgel particles can be observed using an Olympus CKX41SF microscope equipped with an Olympus SC30 frame grabber. Pictures are taken at different magnification to visually inspect the shape of the particles. The particle size and shape of the microgels are assessed by dynamic image analysis with the XPT-C (PS-Prozesstechnik GmbH, Basel, Switzerland). The microgels are kept in suspension in their hardening bath, and then flowed (n=1000) in front of a near-infrared light source. The particle size is expressed by the Waddle disk diameter, which is the diameter of a disc with the same area as the detected particle.

Besides the API the microgel particles contain a polymer and preferably a gelling agent. The polymer must be gelled in order to effectively protect the API against the environment in the gastrointestinal tract. Suitable gel-forming polymers are for example chitosan, chitosan derivatives, polyacrylic acids, alginate, carrageenan, gum Arabic, gellan gum, proteins, xanthan gum, gelatin, agar, pectin, hyaluronic acid and its salts. These polymers can be used alone or in combination of two or more of these polymers.

Suitable chitosan derivatives are alkylated and/or carboxyalkylated and/or PEGylated chitosans wherein the hydroxyl and/or amino groups, preferably the amino groups may be partially or totally alkylated and/or carboxyalkylated. Suitable hydrocarbon groups in the alkylated and/or carboxyalkylated chitosans are saturated, unsaturated or aromatic hydrocarbon groups, such as alkyl or alkenyl groups, in particular those having 1 to 24, preferably 1 to 10, more preferably 1 to 6 carbon atoms. As aromatic hydrocarbon group phenyl is suitable. The hydrocarbon groups may be substituted with one or more substituents, such as hydroxyl, amino and carboxy. A preferred alkyl group is methyl and a preferred carboxyalkyl group is carboxymethyl. Other suitable residues are for example phthalate, succinate and fatty acid esters, such as linoleate and oleate. As chitosan derivatives N-trimethyl chitosan and carboxymethyl chitosan (mono-N-carboxymethylated chitosan) can be exemplified. As proteins albumin and whey proteins can be exemplified. A preferred gel-forming polymer is carboxymethyl chitosan.

Gelling of the polymer is preferably obtained in the presence of a divalent and/or trivalent metal ion as gelling agent. For example, sodium alginate gels in the presence of divalent or trivalent metal ions, such as Ca²⁺, due to the formation of Ca-alginate.

Suitable divalent metal ions are for example Ca²⁺, Mg^{2*}, Zn²⁺, Ba²⁺ and Cu²⁺. A suitable trivalent metal ion is for example Al³⁺. Ca²⁺, Mg²⁺ and Zn²⁺ are preferred and Ca²⁺ being most preferred. Other suitable gelling agents are for example tripolyphosphate, citric acid, phytic acid and glutaraldehyde. Mixtures of two or more of these ions or substances may also be used. The ions are provided in the liquid non-aqueous, in particular lipid composition by dissolving suitable salts (or for example their hydrates) in the composition, for example CaCl₂ or one of its hydrates, such as CaCl₂ dihydrate.

Some polymers can be gelled for example by differences in temperature or pH. In these cases it is not necessary that the microgel particles contain a gelling agent.

The microgel particles may contain further ingredients, such as water, glycerol, buffering agents and the like.

The multi-particulate drug delivery system of the capsule of the invention may contain one type or two or more different types of microgel particles being dispersed in the liquid non-aqueous, in particular lipid composition. If two or more different types of microgel particles are present, these types can for example differ in their size, form, API(s), polymer(s) and/or other ingredients. Different types of microgel particles can also differ in their function, such as sustained, delayed or immediate release microgel particles.

The liquid non-aqueous composition, which does not contain any paraffin oil and is used for dispersing the microgel particles is not particularly limited. However, it should be pharmaceutically acceptable and it should not interfere with usual capsule materials, such as gelatin, hydroxypropyl methylcellulose or starch. The composition should be liquid at 50°C or below, preferably at 40°C or below, more preferably at 30°C or below and most preferably at 25°C or below, such as at room temperature (23°C). Preferably, the liquid composition comprises at least one glyceride (i.e. an ester formed from glycerol and an organic acid; here also referred to as "glyceride derivative") or an alcohol, such as ethanol.

The glyceride can for example be selected from mono-, di- and triglycerides (i.e. the glycerol ester is formed with one, two or three organic acids, respectively) of saturated and/or unsaturated C₂₋₂₈ carboxylic acids, preferably saturate and/or unsaturated C₂₋₂₂ carboxylic acids, more preferably saturated and/or unsaturated C₂₋₂₀ carboxylic acids. In a further preferred embodiment, the glyceride is selected from mono- and diglycerides of saturated C₆₋₁₂ carboxylic acids, preferably saturated C₈₋₁₀ carboxylic acids, or unsaturated C₁₆₋₂₀ carboxylic acids, preferably unsaturated C₁₈ carboxylic acids. Monoglycerides of said carboxylic acids are particularly preferred. Di- and triglycerides may contain two or three different carboxylic acid residues. The glyceride may further contain other residues, such as polyethyleneoxide residues, in particular one polyethylene oxide residue, such as macrogol 3-20, preferably macrogol 3-15, more preferably macrogol 4-10, such as macrogol-4, -5, -6, -7, -8 or -9, in particular macrogol-6 and macrogol-8.

Suitable glycerides are for example mono-, di- and triglycerides containing acetate, caprylate, caprylocaprate, caprate, stearate, oleate, laurate, linolenate and/or linoleate residues. Examples of suitable glycerides are glyceryl monolinoleate, like Maisine® 35-1, decanoyl octanoyl glycerides, like Imwitor® 742, glyceryl monocaprate, like Capmul® MCM C10 EP, glyceryl monocaprylocaprate, like Capmul® MCM EP, glyceryl monocaprylate, like Capmul® MCM C8 EP, glyceryl tricaprylate, like Captex® 8000, glyceryl tricaprate, like Captex® 1000, glyceryl tricaprylocaprate, like Miglyol® 812, caprylocaprate macrogol-6 glycerides, like Acconon® CC-6, caprylocaprate macrogol-8 glycerides, like Acconon® MC-8 EP/NF, linoleoyl macrogol-6 glycerides, like Labrafil® M2125CS, and oleoyl macrogol-6 gylcerides, like Labrafil® M1944CS.

In a further embodiment the glyceride can be selected from polyglyceryls (i.e. polymers wherein the glycerol is bound to other glycerol groups and wherein the furthermost glycerols may form an ester with organic acids or may be substituted by other residues, such as polyethylene oxide residues). As organic acids forming esters and polyethylene oxide residues those described above with respect to the mono-, di- and triglycerides are preferred.

Suitable polyglyceryls are for example polyglyceryl-3 dioleate, like Plurol Oleique CC 497, polyglyceryl-6 oleate, polyglyceryl-10 distearate, polyglyceryl-10 isostearate and polyglyceryl-10 laurate.

It was found that the presence of PEGylated gylceride derivatives and in particular the presence of macrogol-6 gylcerides in the liquid non-aqueous composition surprisingly increased the encapsulation efficacy during preparation of the microgel particles as well as the stability of the microgel particles with respect to the stabilization of proteins contained in the micogel particles under storage conditions compared to microgel particles being prepared using a liquid non-aqueous composition containing glycerides without polyethylene oxide residues.

It has surprisingly been found by the present inventors that the hardening bath which is used for gelling the gel-forming polymer in the manufacture of the microgel particles can be used as the liquid non-aqueous, in particular lipid composition for dispersing the microgel particles. This finding makes it possible for the first time to use the microgel particles in the preparation of the capsule of the invention without the requirement of an intermediate separation of the microgel particles from the hardening bath and drying of the thus obtained microgel particles. However, depending on the liquid non-aqueous, in particular lipid composition and in particular depending on the glyceride used in such composition the salt of a divalent or trivalent metal ion used for gelling the gel-forming polymer may be hardly soluble or even insoluble in the composition. The present inventors found that in such case the solubility of the salt in the liquid non-aqueous, in particular lipid composition can be sufficiently increased by the addition of a co-solvent for dissolving the divalent and/or trivalent metal ion salt in the composition. As co-solvent diethylene glycol monoethylether, ethanol, 2-pyrrolidone, caprylic acid, propylene glycol and N-methlyl-2-pyrrolidone can be exemplified. Diethylene glycol monoethylether (Transcutol® HP) and ethanol being preferred, diethylene glycol monoethylether being most preferred.

It was found that the presence of diethylene glycol monoethylether as co-solvent increases the encapsulation efficacy and stability of proteins contained in the microgel particles during storage compared to the presence of ethanol as co-solvent.

The non-aqueous, in particular lipid composition may further comprise a filler. Suitable fillers are for example polyethylene glycol, such as PEG600, propylene carbonate and natural oils, such as peppermint oil.

It was found that the presence of polyethylene glycol, such as PEG 600, and propylene carbonate significantly increases the encapsulation efficacy during preparation of the microgel particles compared to the presence of natural oils as a filler in the non-aqueous composition.

The non-aqueous, in particular lipid composition may comprise further ingredients, such as glycerol or known permeation enhancers.

The amounts of the ingredients of the non-aqueous, in particular lipid composition can be varied in wide ranges. For example, the composition may contain a weight ratio of co-solvent:glyceride:filler in the ranges of 1.5:8.5:0 to 1.5:0.1:8.4. Preferably, the amount of co-solvent is sufficient to increase the solubility of the divalent or trivalent metal ion salt, such as CaCl₂, in the composition to an extent that 1 to 10wt.%, preferably 2 to 7 wt.% of the salt based on the total weight of the composition can be dissolved in this composition. The non-aqueous, in particular lipid composition can suitably contain at least 15 wt.%, preferable at least 20 wt.% of the co-solvent, based on the total weight of the non-aqueous, in particular lipid composition.

The present invention further relates to a process of preparing the above described capsule containing the multi-particulate drug delivery system. This process comprises the steps of
a) providing a mixture of a gel-forming polymer and an active pharmaceutical ingredient,
b) forming the mixture obtained in step a) into microdroplets,
c) gelling the microdroplets obtained in step b) in a liquid non-aqueous composition to form microgel particles dispersed in the liquid non-aqueous composition, wherein the gelling preferably is carried out in a liquid lipid composition, which does not contain any paraffin oil and further comprising the step of filling the dispersion obtained in step c) into the capsule without isolating the microgel particles from the liquid composition.

In step a) of the above process, the gel-forming polymer and the active pharmaceutical ingredient are mixed. Generally, this mixing is carried out in the presence of water to form a solution of the gel-forming polymer. The amount of the gel-forming polymer is not particularly limited and it depends on the viscosity of the obtained solution. If the viscosity becomes high, it will be difficult to form the mixture into microdroplets. Therefore, low viscosity solutions are preferred. For example, when carboxymethyl chitosan is used as gel-forming polymer, the solution can advantageously contain 1 to 8 wt.%, preferably 2 to 6 wt.%, most preferably about 5 wt.% of the gel-forming polymer based on the total weight of the obtained mixture. The solution can also contain a mixture of two or more gel-forming polymers.

The mixture can comprise further ingredients, such as glycerol. The amount of glycerol can be for example in the range of 1 to 70 wt.%, preferably in the range of 20 to 70 wt.%, more preferably in the range of 30 to 60 wt.% and most preferably in the range of 40 to 55 wt.% based on the total weight of the mixture.

In a further preferred embodiment the mixture additionally contains one or more buffering agents such as Tris (tris(hydroxymethyl)aminomethane) or PBS (phosphate buffer saline).

In step b) of the above process, the mixture obtained in step a) is formed into microdroplets. Formation of microdroplets can be carried out by any method known to the person skilled in the art. Various methods are for example described in M. Whelehan, et al., in Journal of Microencapsulation, 2011; 28(8): 669-688. Mechanical techniques are the most common types of mechanisms used for producing microparticles for medical applications. They are based on the principle of generating a droplet from a polymer extruded through a nozzle and work using mechanical means (i.e. cutting or vibration forces) to increase the normal dripping process at the orifice, or they break up the extruded liquid stream produced by the polymer when it is passed through the nozzle. Some of the main mechanical technologies for forming a fluid dispersion into droplets and subsequent conversion into gel particles are: coaxial airflow, electrostatic extrusion, rotating disc, jet-cutting, spray-drying, vibrating nozzle and prilling. All these methods are known to a person skilled in the art and suitable devices are commercially available. In the process of the present invention step b) preferably is carried out by using vibrating nozzle technique or prilling.

In step c) of the above process, the microdroplets obtained in step b) are gelled to form microgel particles. Generally, after production, the droplets are immediately solidified to microgel particles (spheres or capsules) by chemical means using a gelling agent, such as chemical cross-linking (e.g. chitosan with glutaraldehyde), coacervation/precipitation (e.g. mixtures of chitosan, gellan, carrageenan using physicochemical properties like transition temperature or pH) or ionic gelation (e.g. chitosan or alginate and divalent or trivalent metal ions). Ionic gelation is preferred in the process of the present invention.

The gelling in step c) is carried out in a liquid non-aqueous, preferably lipid composition, which does not contain any paraffin oil. It was found by the present inventors that it is possible to dissolve a sufficient amount of divalent and trivalent metal ions in a liquid non-aqueous composition for carrying out the ionic gelation of the microparticles directly in the final aqueous, preferably lipid composition or in a composition which can be converted into the final aqueous, preferably lipid composition without the requirement of isolating the microgel particles from the hardening bath. For example, the hardening bath can consist of only co-solvent or co-solvent and glyceride and either glyceride and filler or only filler are added after the formation of the microgel particles. Preferably the liquid lipid composition is used as hardening bath.

This has the advantage that the prior art gelling step, which was always carried out in an aqueous solution of the salt, can be omitted thereby saving time and costs. As a consequence, the obtained mixture of microgel particles and liquid composition can be immediately used for further processing without the requirement of any purification or drying steps.

For example, the formed microdroplets fall into the gelling or hardening bath which consists of the liquid non-aqueous composition. As soon as the microdroplets are immersed in the liquid composition, the gel-forming polymer gels in the presence of the divalent or trivalent metal ions thereby forming microgel particles which are simultaneously dispersed in the liquid composition.

If the microdroplets are gelled in the liquid non-aqueous composition, the obtained dispersion is processed into the capsule without isolating the microgel particles from the liquid composition. For example, the dispersion can be filled into capsules either immediately after its preparation, or after adding further ingredients or the dispersion can be stored for some time and can then be further processed, filled into capsules. In any case, expensive and time-consuming isolation, washing and drying steps can be avoided.

Furthermore, it has surprisingly been found that as further advantage of the process of the present invention, in particular when the microgel particles are formed by gelling the gel-forming polymer of the microdroplets in the liquid non-aqueous composition, the microgel particles exhibit a higher mechanical stability, an increased encapsulation efficiency and an increased storage stability compared to microgel particles being prepared by gelling in an aqueous hardening bath. Thus, the microgel particles and the multi-particulate drug delivery system of the capsule obtained by the process of the present invention also differs in its physical properties from a comparable drug delivery system wherein the microgel particles have been prepared according to the prior art processes, isolated, washed and dried before being further processed.

The multi-particulate drug delivery system is further processed to obtain a suitable unit dosage form, the capsule. Suitable pharmaceutical capsules are for example hard or soft shell capsules. Suitable capsule materials are for example gelatin, hydroxypropyl methylcellulose and starch.

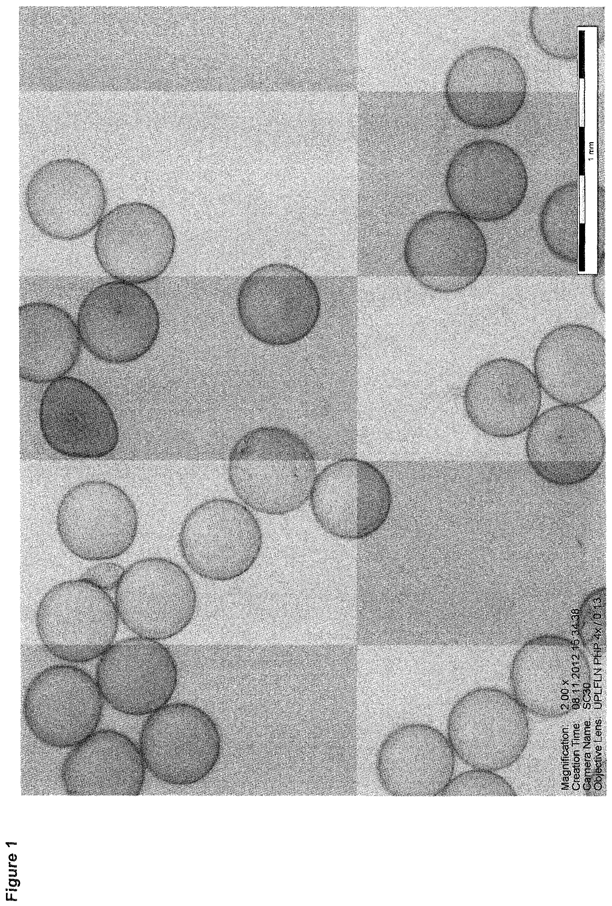

Figure 1 shows microgel particles obtained according to the present invention.
Figure 2 shows the encapsulation efficacy using different hardening baths.
Figure 3 shows the leakage from microgel particles obtained with different hardening baths.
Figure 4 shows the release of BSA from microgel particles obtained with different hardening baths.

The invention will now be further illustrated by the examples which are not intended to be construed as being limiting.

### Example 1

Two polymeric solutions containing the following ingredients were prepared:

| Polymeric solution 1 | | |
|---|---|---|
| Substance | Quantity (g) | Quantity (w/w %) |
| Carboxymethyl chitosan powder | 23.00 (dry) | 4.6 |
| Bovine serum albumin powder 96 % | 12.50 | 2.5 |
| Water | To 500 | 92.9 |

| Polymeric solution 2 | | |
|---|---|---|
| Substance | Quantity (g) | Quantity (w/w %) |
| Carboxymethyl chitosan powder | 17.50 (dry) | 3.50 |
| Bovine serum albumin powder 96 % | 12.50 | 2.50 |
| Water | To 500 | 94.00 |

In both cases, carboxymethyl chitosan and bovine serum albumin were weighed and mixed together under stirring from 300 rpm to 700 rpm. The blend was then filtered on glass microfiber filters with opening of 1 µm to 5 µm under vacuum. The final composition rested overnight (at least 8 hours) to remove any air bubble in the formulation. The solution was then used with the vibrating nozzle unit.

From these polymeric solutions microdroplets were prepared using an Encapsulator BIOTECH from EncapBioSystems with the following settings:

| Parameter | Value |
|---|---|
| Frequency | 1000 Hz - 1500 Hz |
| Amplitude | 2 - 7 |
| Light intensity | 1 - 9 |
| Electrode | 500 V - 1700 V |
| Pump | 2.8 mL/min - 3.5 mL/min |
| Stirring speed | 80 % - 100 % |

For gelling of the microdroplets the following hardening baths were used:

| Hardening bath 1 | | |
|---|---|---|
| Substance | Quantity (g) | Quantity (w/w %) |
| Transcutol HP liquid | 160 | 38.10 |
| Capmul MCM-C8 EP liquid | 120 | 28.57 |
| Propylene carbonate liquid | 120 | 28.57 |
| Calcium chloride dihydrate powder | 20 | 4.76 |

Transcutol HP, Capmul MCM-C8 EP, and propylene carbonate were weighed and mixed together under stirring from 500 rpm to 900 rpm. Calcium chloride dihydrate was weighed and added to the blend under stirring from 800 rpm to 1100 rpm until the powder was completely dissolved. The blend was then used as hardening bath.

| Hardening bath 2 | | |
|---|---|---|
| Substance | Quantity (g) | Quantity (w/w %) |
| Ethanol liquid | 120 | 28.92 |
| Capmul MCM EP liquid | 140 | 33.73 |
| Propylene carbonate liquid | 140 | 33.73 |
| Calcium chloride dihydrate powder | 15 | 3.62 |

Hardening bath 2 was prepared basically in the same manner as hardening bath 1, except that Capmul MCM EP was heated to 40°C before being used.

Using polymeric solution 1 microgel particles having the following parameters were obtained:

| Parameter | Hardening bath 1 | Hardening bath 2 |
|---|---|---|
| Encapsulating efficiency | 90 - 95 % | 95 - 100 % |
| Median particle size | 300 - 330 µm | 280 - 300 µm |
| Leakage (after 4 weeks) | < 5 % | < 5 % |

### Example 2

A polymeric solution was prepared by dissolving 4.76 % (dry substance; w/v) solution of carboxymethyl chitosan in demineralized water. An appropriate amount of bovine serum albumin (BSA) was added to obtain 2.5 % (w/v) solution. The solution was then stored in a glass brown bottle at +4°C, and it was allowed to reach room temperature before each use.

Seven hardening baths were prepared, each comprising 4.76 % (w/w) of calcium chloride. Their compositions are listed in the below table:

| Hardening bath | Composition | Ratio (as weight) | Quantity* (w/w) |
|---|---|---|---|
| HBW (comparative) | | | |
| | Water | 1 | 95.24% |
| EtOH | Ethanol | 1 | 95.24% |
| HBA | Transcutol® HP | 1 | 95.24% |
| HBB | Transcutol® HP | | 31.74% |
| | Imwitor 742 | 1 : 1 : 1 | 31.74% |
| | Propylene carbonate | | 31.74% |
| HBC | Transcutol® HP | | 47.62% |
| | Capmul® MCM-C8 EP | 2 : 1 : 1 | 23.81% |
| | Propylene carbonate | | 23.81% |
| HBD | Transcutol® HP | | 47.62% |
| | Capmul® MCM EP | 2 : 1 : 1 | 23.81% |
| | PEG 600 | | 23.81% |
| HBE | Transcutol® HP | 1 : 1 : 1 | 31.74% |
| | Labrafil® M2125CS | | 31.74% |
| | Peppermint oil | | 31.74% |

| | | | |
|---|---|---|---|
| * after adding of calcium chloride to 4.76% of total | | | |

Hardening bath HBW was used as comparative.

The microgels were prepared by means of the vibrating nozzle technique on the Encapsulator Biotech (EncapBioSystems Inc., Greifensee, Switzerland; this product is now commercialised by Buchi Labortechnik AG, Flawil, Switzerland). The polymeric solution was loaded in 20 mL Omnifix® plastic syringes (B. Braun Melsungen AG, Melsungen, Germany). The polymeric solution was then pumped through a 150 µm stainless steel nozzle, at a nominal flow rate of 3.10 mL/min (3.79 g/min of polymeric solution) by applying a frequency of 1240 Hz, and by setting the electrode ring to 1500 V. The fall distance was of ∼13 cm into 100 mL of hardening bath, which was stirred at 400 rpm. The microgels were then left standing in the hardening baths for 20 minutes before further analyses.

Size and form of the microgel particles were determined as described above. The results are summarized in the following table:

| Hardening bath | Particle size (µm) | | Elongation |
|---|---|---|---|
| | D₅₀ | D₉₀ | factor |
| HBW (comparative) | | | |
| | 410.0 | 473.7 | 2.9 |
| EtOH | 292.9 | 346.4 | 2.1 |
| HBA | 300.5 | 347.8 | 2.0 |
| HBB | 320.7 | 364.1 | 1.8 |
| HBC | 319.9 | 364.1 | 1.8 |
| HBD | 332.7 | 400.3 | 2.0 |
| HBE | 316.0 | 355.6 | 1.7 |

For BSA quantification and analysis, 5 g aliquots of microgel were passed through a 125 µm opening stainless steel sieve, and then washed with water, ethanol, and again with water. The microgels were diluted to 50 mL in a volumetric flask (nominal BSA concentration of 2.5 mg/mL) with phosphate buffer saline pH 6.8 and allowed to release all their content over 72 hours at room temperature under constant stirring. The samples were subsequently stored at +4°C. Before each use, the required amount of solution was centrifuged at 10 000 rpm for 10 minutes in a 5415C centrifuge (Eppendorf GmbH, Leipzig, Germany) and then filtered through Titan3 nylon filters 0.45 µm (SMI-LabHut Ltd, Maisemore, UK).

### Encapsulation efficiency (EE) of BSA in microgels

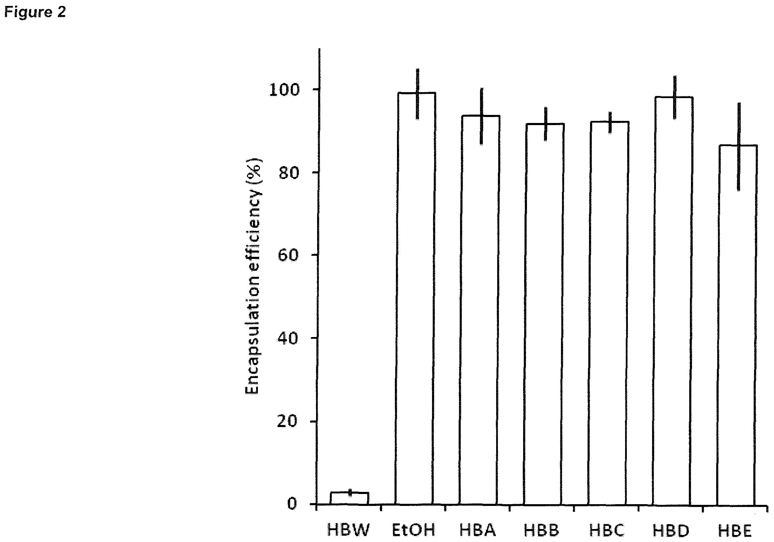

Aliquots of the BSA solutions were diluted with more buffer solution to a final nominal BSA concentration of 1.5 mg/mL. The protein content was measured by means of *DC*™ Protein Assay, which is based on the Lowry protein assay (Lowry et al., 1951; Peterson, 1979), according to the protocol supplied by the company. The protein content was measured on a Jasco V-630 UV-Vis spectrophotometer (Jasco Inc., Easton, US) at 750 nm in 1 cm optical path Plastibrand® disposable semi-micro PMMA cuvettes (Brand GmbH + CO KG, Wertheim, Germany). The encapsulation efficiency is expressed in percentage as the ratio between the BSA encapsulated and the amount of BSA present in the polymeric solution. The results are shown in Figure 2.

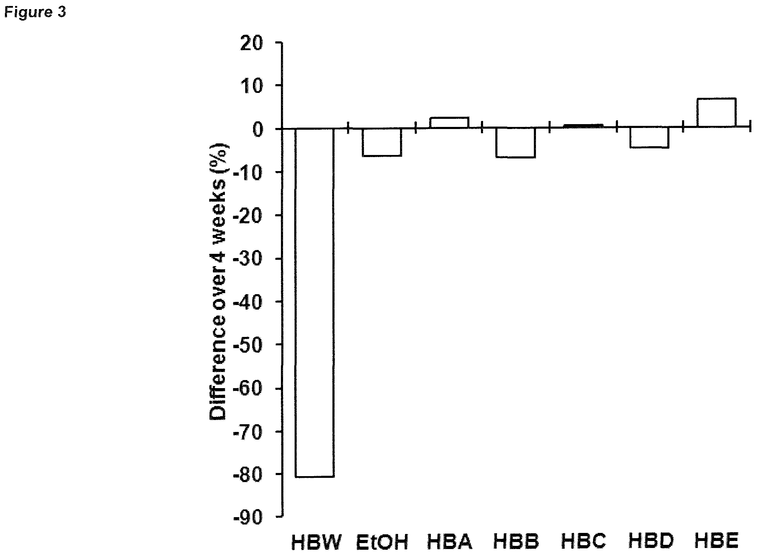

The BSA leakage from the polymeric matrix while in the hardening bath was also tested by measuring the EE over time. The suspended microgels were kept at 25°C over a period of one month in amber bottles; the EE was measured at weeks 1, 2, 3, and 4. The leakage is expressed in percentage as the EE at a given time point compared to the time zero EE. The results are shown in Figure 3.

### In vitro release of BSA from microgels

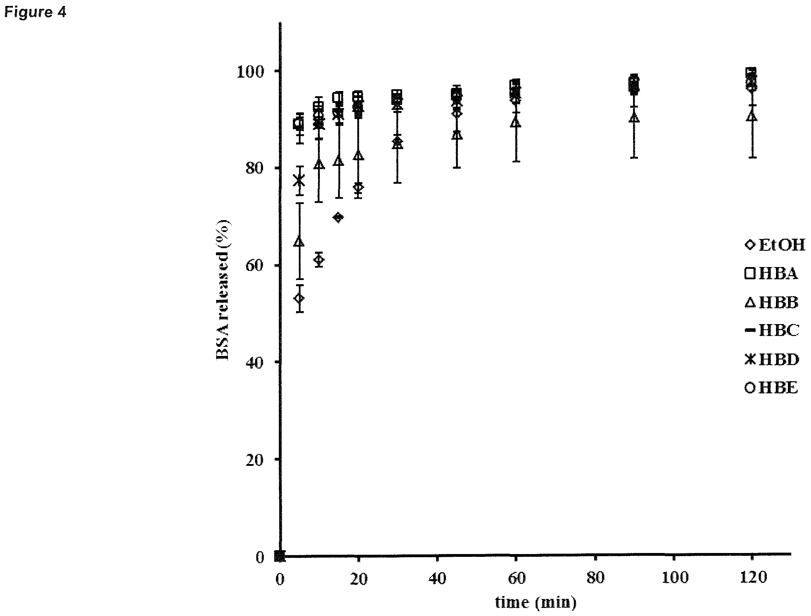

The *in vitro* release of the microgels was tested on an Erweka DT 600 (Erweka GmbH, Hausenstamm, Germany) equipped with paddle, as described in the European Pharmacopoeia (2007). Each dissolution vessel (n = 3) was filled with 500 mL of phosphate buffer saline pH 6.8, which was heated at 37 ± 0.5°C and stirred at 50 rpm. Then, washed BSA-loaded microgels were added to a nominal total content of 15 mg of BSA per vessel. At different time points (5, 10, 15, 20, 30, 45, 60, 90, and 120 minutes) a 1 mL sample was drawn from the release medium and filtered through nylon filters 0.45 µm; the corresponding volume of release medium drawn was then compensated with fresh buffer. A final aliquot was taken after 12 hours at 100 rpm, to obtain the BSA release from the microgels at equilibrium. The protein content of these samples was measured by means of Micro BCA™ Protein Assay Kit (Thermo Fisher Scientific Inc., Rockford, US), which is based on the bicinchoninic acid protein assay (Brown et al., 1989; Kessler and Fanestil 1986; Smith et al., 1985; Wiechelman et al., 1988), according to the protocol supplied by the company. The prepared samples were loaded in 96-well clear BRANDplates® pureGrade™ (Brand GmbH + CO KG, Wertheim, Germany) and measured in the SpectraMax M2^{e} at λ = 562 nm. The values obtained are expressed as a percentage of the release at equilibrium value. The results are shown in Figure 4.

## Claims

1. A capsule containing a multi-particulate drug delivery system which comprises microgel particles containing an active pharmaceutical ingredient, said microgel particles being dispersed in a liquid non-aqueous composition which does not contain any paraffin oil.

2. The capsule according to claim 1, wherein the microgel particles contain at least one gel-forming polymer selected from the group consisting of chitosan, chitosan derivatives that are alkylated and/or carboxylated and/or PEGylated chitosans wherein the hydroxyl and/or amino groups may be partially or totally alkylated and/or carboxylated, polyacrylic acids, alginate, carrageenan, gum Arabic, gellan gum, xanthan gum, proteins, gelatin, agar, pectin, hyaluronic acid and its salts, and wherein the microgel particles preferably are obtainable by gelling the gel-forming polymer in the presence of a divalent and/or trivalent metal ion.

3. The capsule according to any of the preceding claims, wherein the non-aqueous composition is a lipid composition, preferably wherein the lipid composition comprises at least one glyceride, which more preferably is selected from mono-, di- and triglycerides of saturated and/or unsaturated C₂₋₂₈ carboxylic acids, wherein the monoglycerides may additionally comprise one or two polyethylene oxide residues and the diglycerides may additionally comprise one polyethylene oxide residue.

4. The capsule according to claim 3, wherein the glyceride is selected from mono- and diglycerides of saturated C₆₋₁₂ carboxylic acids or unsaturated C₁₆₋₂₀ carboxylic acids, wherein the monoglycerides may additionally comprise one or two polyethylene oxide residues and the diglycerides may additionally comprise one polyethylene oxide residue.

5. The capsule according to any of the preceding claims, wherein the non-aqueous composition further comprises a co-solvent for dissolving a divalent and/or trivalent metal ion salt in the non-aqueous composition, wherein the co-solvent preferably is selected from diethylene glycol monoethylether, ethanol, 2-pyrrolidone, caprylic acid, propylene glycol and N-methyl-2-pyrrolidone.

6. The capsule according to any of the preceding claims, wherein the non-aqueous composition further comprises a filler selected from polyethylene glycol, propylene carbonate and natural oils.

7. The capsule according to any of the preceding claims, wherein the microgel particles have a particle size distribution D₉₀ of below 1000 µm.

8. The capsule according to any of the preceding claims, wherein the microgel particles have an elongation factor in the range of 1.27 to 2.60 wherein the elongation factor is the max Feret diameter (the linear segment connecting the two perimeter points that are the furthest apart) divided by the Feret equivalent rectangular short side (the shortest side of the rectangle with the same area as the particle and the longest side equal in the length to the max Feret diameter).

9. Process of preparing the capsule according to any of claims 1-8, comprising the steps of
a) providing a mixture of a gel-forming polymer and an active pharmaceutical ingredient,
b) forming the mixture obtained in step a) into microdroplets, preferably by using vibrating nozzle technique or prilling,
c) gelling the microdroplets obtained in step b) in a liquid non-aqueous composition to form microgel particles dispersed in the liquid non-aqueous composition, wherein the gelling preferably is carried out in a liquid lipid composition, which does not contain any paraffin oil, and
further comprising the step of filling the dispersion obtained in step c) into the capsule without isolating the microgel particles from the liquid composition.

## Patentansprüche

1. Kapsel, enthaltend ein multipartikuläres Arzneimittelabgabesystem, das Mikrogelpartikel umfasst, die einen aktiven pharmazeutischen Inhaltsstoff enthalten, wobei die Mikrogelpartikel in einer flüssigen, nicht wässerigen Zusammensetzung dispergiert sind, die kein Paraffinöl enthält.

2. Kapsel nach Anspruch 1, wobei die Mikrogelpartikel mindestens ein gelbildendes Polymer enthalten, das ausgewählt ist aus der Gruppe, bestehend aus Chitosan, Chitosanderivaten, die alkylierte und/oder carboxylierte und/oder PEGylierte Chitosane sind, wobei die Hydroxyl- und/oder Aminogruppen teilweise oder vollständig alkyliert und/oder carboxyliert sein können, Polyacrylsäuren, Alginat, Carrageenan, Gummiarabikum, Gellan, Xanthan, Proteinen, Gelatine, Agar, Pektin, Hyaluronsäure und ihren Salzen, und wobei die Mikrogelpartikel vorzugsweise durch Gelierung des gelbildenden Polymers in Gegenwart eines zweiwertigen und/oder dreiwertigen Metallions erhältlich sind.

3. Kapsel nach einem der vorhergehenden Ansprüche, wobei die nicht wässerige Zusammensetzung eine Lipidzusammensetzung ist, wobei die Lipidzusammensetzung vorzugsweise mindestens ein Glycerid umfasst, das stärker bevorzugt ausgewählt ist aus Mono-, Di- und Triglyceriden von gesättigten und/oder ungesättigten C₂₋₂₈-Carbonsäuren, wobei die Monoglyceride zusätzlich einen oder zwei Polyethylenoxidreste umfassen können und die Diglyceride zusätzlich einen Polyethylenoxidrest umfassen können.

4. Kapsel nach Anspruch 3, wobei das Glycerid ausgewählt ist aus Mono- und Diglyceriden von gesättigten C₆₋₁₂-Carbonsäuren oder ungesättigten C₁₆₋₂₀-Carbonsäuren, wobei die Monoglyceride zusätzlich einen oder zwei Polyethylenoxidreste umfassen können und die Diglyceride zusätzlich einen Polyethylenoxidrest umfassen können.

5. Kapsel nach einem der vorhergehenden Ansprüche, wobei die nicht wässerige Zusammensetzung ferner ein Co-Lösungsmittel zum Auflösen eines zweiwertigen und/oder dreiwertigen Metallionensalzes in der nicht wässerigen Zusammensetzung umfasst, wobei das Co-Lösungsmittel vorzugsweise ausgewählt ist aus Diethylenglycolmonoethylether, Ethanol, 2-Pyrrolidon, Caprylsäure, Propylenglycol und N-Methyl-2-pyrrolidon.

6. Kapsel nach einem der vorhergehenden Ansprüche, wobei die nicht wässerige Zusammensetzung ferner einen Füllstoff umfasst, der ausgewählt ist aus Polyethylenglycol, Propylencarbonat und natürlichen Ölen.

7. Kapsel nach einem der vorhergehenden Ansprüche, wobei die Mikrogelpartikel eine Teilchengrößenverteilung D₉₀ unter 1000 µm aufweisen.

8. Kapsel nach einem der vorhergehenden Ansprüche, wobei die Mikrogelpartikel einen Elongationsfaktor im Bereich von 1,27 bis 2,60 aufweisen, wobei der Elongationsfaktor der maximale Feret-Durchmesser (lineares Segment, das die beiden Umfangspunkte verbindet, die am weitesten voneinander entfernt sind) geteilt durch Feret-Äquivalent-Rechtecks-Kurzseite (kürzeste Seite des Rechtecks mit derselben Fläche wie das Partikel und längsten Seite mit der gleichen Länge wie der maximale Feret-Durchmesser) ist.

9. Verfahren zur Herstellung der Kapsel nach einem der Ansprüche 1 - 8, umfassend die Schritte:
a) Bereitstellen eines Gemisches aus einem gelbildenden Polymer und einem aktiven pharmazeutischen Inhaltsstoff,
b) Formen des in Schritt a) erhaltenen Gemisches zu Mikrotröpfchen, vorzugsweise unter Anwendung einer Vibrationsdüsentechnik oder Prillen,
c) Gelierung der in Schritt b) erhaltenen Mikrotröpfchen in einer flüssigen, nicht wässerigen Zusammensetzung unter Bildung von Mikrogelpartikeln, die in der flüssigen, nicht wässerigen Zusammensetzung dispergiert sind, wobei die Gelierung vorzugsweise in einer flüssigen Lipidzusammensetzung durchgeführt wird, die kein Paraffinöl enthält, und
ferner umfassend den Schritt des Füllens der in Schritt c) erhaltenen Dispersion in die Kapsel, ohne die Mikrogelpartikel von der flüssigen Zusammensetzung zu isolieren.

## Revendications

1. Capsule contenant un système d'administration de médicament multi particulaire qui comprend des particules de micro-gel contenant un ingrédient pharmaceutique actif, lesdites particules de micro-gel étant dispersées dans une composition liquide non aqueuse qui ne contient aucune huile de paraffine.

2. Capsule selon la revendication 1, dans laquelle les particules de micro-gel contiennent au moins un polymère gélifiant choisi dans le groupe constitué par le chitosane, les dérivés du chitosane qui sont alkylés et/ou carboxylés et/ou les chitosanes PEGylés, dans laquelle les groupes hydroxyle et/ou amino pouvant être partiellement ou totalement alkylés et/ou carboxylés, l'acides polyacryliques, l'alginate, la carraghénane, la gomme arabique, la gomme gellane, la gomme xanthane, les protéines, la gélatine, l'agar, la pectine, l'acide hyaluronique et ses sels, et où les particules de micro-gel peuvent de préférence être obtenues par la gélification du polymère gélifiant en présence d'un ion métallique divalent et/ou trivalent.

3. Capsule selon l'une quelconque des revendications précédentes, dans laquelle la composition non aqueuse est une composition lipidique, de préférence dans laquelle la composition lipidique comprend au moins un glycéride, qui est plus préférablement choisi parmi les mono-, di- et triglycérides d'acides carboxyliques saturés et/ou insaturés en C₂₋₂₈, dans laquelle les monoglycérides peuvent comprendre en outre un ou deux résidus oxyde polyéthylène et les diglycérides peuvent également comprendre un résidu oxyde polyéthylène.

4. Capsule selon la revendication 3, dans laquelle la glycéride est choisi parmi les mono- et diglycérides d'acides carboxyliques saturés en C₆₋₁₂ ou d'acides carboxyliques insaturés en C₁₆₋₂₀, dans laquelle les monoglycérides peuvent comprendre en outre un ou deux oxydes de polyéthylène et les diglycérides peuvent en outre contenir un résidu d'oxyde de polyéthylène.

5. Capsule selon l'une quelconque des revendications précédentes, dans laquelle la composition non aqueuse comprend en outre un co-solvant pour dissoudre un sel d'ion métallique divalent et/ou trivalent dans la composition non aqueuse, dans laquelle le co-solvant est de préférence choisi parmi le diéthylène glycol monoéthyléther, l'éthanol, la 2-pyrrolidone, l'acide caprylique, le propylène glycol et la N-méthyl-2-pyrrolidone.

6. Capsule selon l'une quelconque des revendications précédentes, dans laquelle la composition non aqueuse comprend en outre une charge choisie parmi le polyéthylène glycol, le carbonate de propylène et les huiles naturelles.

7. Capsule selon l'une quelconque des revendications précédentes, dans laquelle les particules de micro-gel ont une distribution granulométrique D₉₀ inférieure à 1000 µm.

8. Capsule selon l'une quelconque des revendications précédentes, dans laquelle les particules de micro-gel ont un facteur d'allongement dans la plage de 1,27 à 2,60, dans laquelle le facteur d'allongement est le diamètre maximal de Feret (segment linéaire reliant les deux points périphériques les plus éloignés) divisé par le côté court rectangulaire équivalent de Feret (le côté le plus court du rectangle ayant la même surface que la particule et le côté le plus long dont la longueur est égale au diamètre maximal de Feret).

9. Procédé de préparation de la capsule selon l'une quelconque des revendications 1 à 8, comprenant les étapes consistant à
a) fournir un mélange d'un polymère gélifiant et d'un ingrédient pharmaceutique actif,
b) former le mélange obtenu à l'étape a) en microgouttelettes, de préférence en utilisant la technique de la buse vibrante ou du grenolage,
c) gélifier les microgouttelettes obtenues à l'étape b) dans une composition liquide non aqueuse pour former des particules de micro-gel dispersées dans la composition liquide non aqueuse, où la gélification est effectuée de préférence dans une composition liquide lipidique, qui ne contient aucune huile de paraffine, et
comprenant en outre l'étape de remplissage de la dispersion obtenue dans l'étape c) dans la capsule sans isoler les particules de micro-gel de la composition liquide.
